# EUROPÄISCHE PATENTANMELDUNG

(11) **EP 0 794 195 A2**
(43) Veröffentlichungstag der Anmeldung: **10.09.1997**
(21) Anmeldenummer: 97100222.5
(22) Anmeldetag: 09.01.1997
(51) Int. Cl.: C07K 16/00

(54) **Antikörper BV10A4H2**

(30) Priorität: 07.03.1996 DE 19608769
(71) Anmelder: Eberhard-Karls-Universität Tübingen Universitätsklinikum, 72076 Tübingen (DE)
(72) Erfinder: Bühring, Hans Jörg, Dr., 72076 Tübingen (DE)
(74) Vertreter: Otten, Hajo, Dr.-Ing.

(57) **Zusammenfassung**

Die Erfindung betrifft einen monoklonalen Antikörper, der spezifisch an das humane FLT3/FLK2-Rezeptorprotein bindet. Die Erfindung betrifft ferner Hybridomzellen, die einen derartigen Antikörper erzeugen, sowie ein Verfahren zur Herstellung solcher Hybridomzellen.

## Beschreibung

Die Erfindung betrifft einen Antikörper gegen den humanen Tyrosin-Kinase-Rezeptor FLT3/FLK2.

Der Tyrosin-Kinase-Rezeptor FLT3/FLK2 spielt eine wichtige Rolle bei der frühen Hämatopoese. Er kommt natürlicherweise in Blutstammzellen und in frühen lymphoiden und myeloischen Blutvorläuferzellen vor und interagiert mit einem Wachstumsfaktor FLT3-Ligand (FL), der insbesondere die Rekrutierung und die Proliferation dieser Zellen stimuliert (Matthews et al., Cell 65:1143, 1991; Small et al., Proc. Natl. Acad. Sci. USA 91:459, 1994; Lyman et al., Blood 83:2795, 1994; Muench et al., Blood 85:963, 1995; Hannum et al., Nature 368:643, 1994; Hirayama et al., Blood 85:1762, 1995).

Der indirekte Nachweis vermittels eines Antikörpers, der spezifisch an den Rezeptor bindet, ist ein sicheres und schnelles Verfahren zum qualitativen und quantitativen Nachweis von membrangebundenen Rezeptoren. Dieser spezifische Antikörper kann entweder direkt oder indirekt markiert sein, wobei diese Markierung dazu benutzt wird, den Antikörper bzw. den damit verbundenen Rezeptor zu identifizieren und zahlenmäßig zu bestimmen. Als Marker kommen hier insbesondere Fluoreszenzfarbstoffe oder radioaktive Stoffe in Betracht.

Ein solcher Antikörper kann auch mit speziellen, therapeutisch wirksamen Reagenzien gekoppelt werden und damit die Möglichkeit einer zellulär zielgerichteten Behandlung bieten, die insbesondere bei Tumorerkrankungen zu fordern ist.

Es wurden bereits Antikörper gegen FLT3/FLK2-Rezeptorprotein beschrieben, diese sind jedoch allesamt spezifisch gegen das Maus-FLT3/FLK2-Rezeptorprotein gerichtet. Für eine Anwendung an menschlichen Zellen sind diese Antikörper deshalb nicht geeignet, denn es fehlt ihnen die diesbezügliche Spezifität.

Der vorliegenden Erfindung liegt daher die Aufgabe zugrunde, einen Antikörper bereitzustellen, der spezifisch an das native, unmodifizierte humane FLT3/FLK2-Rezeptorprotein bindet und der in praktisch unbegrenzter Menge zur Verfügung steht.

Diese Aufgabe wird durch die Bereitstellung eines monoklonalen Antikörpers gelöst, der spezifisch an das native, unmodifizierte humane FLT3/FLK2-Rezeptorprotein bindet. Ein solcher monoklonaler Antikörper wird von Hybridomzellen produziert und freigesetzt, die am 19. Dezember 1995 unter der Nummer DSM ACC2248 bei der Deutschen Sammlung von Mikroorganismen und Zellkulturen GmbH (DSM) nach den Vorschriften des Budapester Vertrags hinterlegt worden sind. Er ist mit der Bezeichnung BV10A4H2 benannt.

Mit dem erfindungsgemäßen Antikörper wurde erstmals ein monoklonaler Antikörper bereitgestellt, der standardisiert reproduzierbar ist und somit potentiell unbegrenzt hergestellt werden kann, und der spezifisch an jeweils ein spezielles extrazelluläres Epitop auf dem humanen Rezeptorprotein FLT3/FLK2 bindet.

Der erfindungsgemäße Antikörper ermöglicht eine gezielte Erkennung und Beeinflussung von Zellen, die eine extrazelluläre Domäne des humanen FLT3/FLK2-Rezeptorproteins aufweisen. Er stellt damit ein bisher einzigartiges und vielseitig einsetzbares Mittel für den Arzt und Forscher dar, um einerseits solche Zellen nachzuweisen, und zwar sowohl in der Zellkultur als auch im Patientenorganismus, und um andererseits diese Zellen ggf. zu manipulieren, entweder durch den Antikörper selbst oder durch daran gekoppelte, spezifische Reagenzien.

Die Erfindung betrifft ferner Hybridomzellen, die einen monoklonalen Antikörper gegen das FLT3/FLK2-Rezeptorprotein erzeugen. Sie umfaßt insbesondere die Hybridomzellen, die unter der Nummer DSM ACC2248 bei der Deutschen Sammlung von Mikroorganismen und Zellkulturen GmbH (DSM) nach dem Budapester Vertrag hinterlegt sind und den Antikörper mit der Bezeichnung BV10A4H2 produzieren.

Darüber hinaus betrifft die Erfindung auch ein Verfahren zur Herstellung von Hybridomzellen, die einen Antikörper gegen das native, unmodifizierte FLT3/FLK2-Rezeptorprotein synthetisieren und freisetzen. Dieses Verfahren umfaßt die im Stand der Technik grundsätzlich geläufigen Schritte, wie sie bspw. von Bühring et al. in Hybridoma 1991, Band 10, Nr. 1, S. 77-78 beschrieben wurden:
1. Immunisierung oder Sensibilisierung eines Tieres, vorzugsweise einer Maus vom Balb/c-Stamm, mit dem Antigen bzw. Immunogen;
2. Gewinnung der antikörperproduzierenden Zellen, vorzugsweise der Milzlymphozyten dieses Tieres;
3. Fusionierung dieser antikörperproduzierenden Zellen mit einer stabilen, immortalisierten Zellinie, vorzugsweise einer Myelomzellinie, zu Hybridomzellen; und
4. Vereinzelung und Vermehrung (Klonierung) solcher Hybridomzellen, die einen Antikörper sezernieren, der an das Antigen bindet.

Das erfindungsgemäß gefundene Verfahren zeichnet sich dadurch aus, daß das Tier mit Zellen der hämatopoetischen Pro-B-Zellinie BV-173 immunisiert wird, die frei bei der DSM unter der Nr. DSM ACC20 verfügbar ist.

Das hat den Vorteil, daß die Zellen dieser Pro-B-Zellinie BV-173 eine starke Expression von FLT3/FLK2-Rezeptorprotein zeigen, wie sich während der zu dem Antikörper BV10A4H2 führenden Versuche zeigte.

Bei der Suche nach Hybridomzellen, die FLT3/FLK2-Rezeptorprotein-spezifische Antikörper produzieren, ist es bevorzugt, wenn nur solche vereinzelten und klonierten Hybridomzellen ausgewählt werden, die Antikörper produzieren, die eine Spezifität für die Zellen der Mauslinie BA/F3 zeigen, die zuvor mit der kompletten DNA-Sequenz für den humanen FLT3/FLK2 transfiziert wurden (BA/F3-huFLT3).

Die Erfindung betrifft auch die Verwendung eines monoklonalen Antikörpers gegen das FLT3/FLK2-Rezeptorprotein zur diagnostischen und/oder therapeutischen Behandlung von Tumoren, insbesondere von malignen hämatopoetischen Zellen wie z.B.: lymphoiden und myeloischen Leukämiezellen.

Es wurde nämlich überraschender Weise gefunden, daß die meisten malignen hämatopoetischen Zellen, wie z.B. lymphoide und myeloische Leukämiezellen, einen vergleichsweise hohen Gehalt an FLT3/FLK2-Rezeptorprotein aufweisen. Ein erfindungsgemäßer Antikörper, der mit einem Nachweismittel, beispielsweise einem radioaktiven Marker, gekoppelt ist, bindet dieses Nachweismittel indirekt an die betreffenden Zellen und ermöglicht damit den direkten Nachweis dieser Zellen, beispielsweise mit röntgendiagnostischen /szintigraphischen Methoden. Damit ist eine sehr frühe Diagnose unter Umständen sogar in-vivo möglich.

In entsprechender Art und Weise kann der Antikörper mit einem therapeutisch wirksamen Mittel gekoppelt sein und dadurch eine direkte und gezielte Beeinflussung oder gar Eliminierung von FLT3/FLK2-Rezeptorprotein tragenden Zellen, insbesondere Leukämiezellen, ermöglichen.

In einer bevorzugten Ausführungsform wird ein Antikörper, der von den unter der Nummer DSM ACC2248 bei der Deutschen Sammlung von Mikroorganismen und Zellkulturen GmbH (DSM) hinterlegten Hybridomzellen produziert und freigesetzt wird, zu einer solchen diagnostischen und/oder therapeutischen Behandlung verwendet.

Um die therapeutische und/oder diagnostische Applikation des erfindungsgemäßen Antikörpers zu erleichtern, kann der Antikörper mit entsprechend geeigneten Hilfssubstanzen in einer pharmazeutischen Zubereitung vermischt sein. Die Erfindung betrifft deshalb auch ein pharmazeutisches Mittel zur diagnostischen und/oder therapeutischen Behandlung von Tumoren, das einen erfindungsgemäßen, an humanes FLT3/FLK2-Rezeptorprotein bindenden Antikörper enthält. Vorzugsweise enthält dieses pharmazeutische Mittel einen Antikörper, wie er von den unter der Nummer DSM ACC2248 bei der Deutschen Sammlung von Mikroorganismen und Zellkulturen GmbH (DSM) hinterlegten Hybridomzellen produziert und freigesetzt wird.

Mit einem erfindungsgemäßen Antikörper können humanes FLT3/FLK2-Rezeptorprotein tragende Zellen aus einer Suspension verschiedenartiger (humaner) Zellen mit den im Stand der Technik bekannten Testverfahren, wie z.B. dem Enzyme linked immunosorbet assay, kurz ELISA, oder dem Radioimmunoassay, kurz RIA, nachgewiesen werden. Die vorliegende Erfindung betrifft deshalb auch einen Kit zum Nachweis von humanem FLT3/FLK2-Rezeptorprotein, der einen monoklonalen Antikörper umfaßt, der spezifisch an das native humane FLT3/FLK2-Rezeptorprotein bindet.

Eine bevorzugte Ausführungsform dieses Kits ist dadurch gekennzeichnet, daß der Kit einen Antikörper umfaßt, wie er von den unter der Nummer DSM ACC2248 bei der Deutschen Sammlung von Mikroorganismen und Zellkulturen GmbH (DSM) hinterlegten Hybridomzellen erzeugt wird.

Im Rahmen der vorliegenden Erfindung wurde überraschenderweise gefunden, daß der monoklonale Antikörper mit der Bezeichnung BV10A4H2, der von den unter der Nummer DSM ACC2248 bei der Deutschen Sammlung von Mikroorganismen und Zellkulturen GmbH (DSM) hinterlegten Hybridomzellen erzeugt wird, an humane Stammzellen bindet.

Die Erfindung betrifft deshalb auch die Verwendung eines erfindungsgemäßen Antikörpers, vorzugsweise des Antikörpers BV10A4H2, zum Nachweis von hämatopoetischen Zellen, sowie einen Kit zum Nachweis von hämatopoetischen Zellen, der einen erfindungsgemäßen Antikörper, vorzugsweise den Antikörper BV10A4H2, enthält. Dadurch ist es möglich, undifferenzierte CD34⁺ Subpopulationen aufzutrennen und für funktionelle Analysen Zellen aus dem Knochenmark zu selektieren und aufzureinigen.

Weitere Vorteile ergeben sich aus der nachstehenden Beschreibung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird im folgenden anhand von Anwendungs- und Ausführungsbeispielen unter Heranziehung der Figuren näher erläutert.

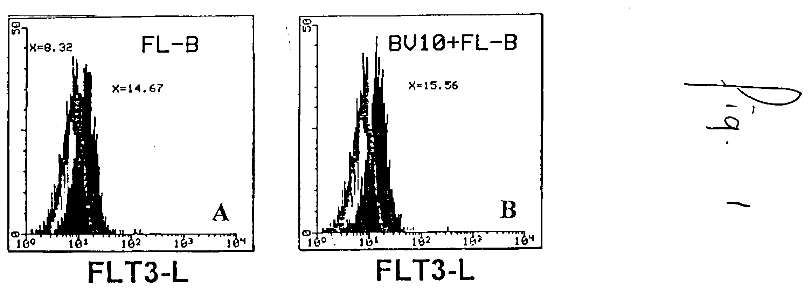

- Fig. 1: umfaßt das Histogramm A einer Durchflußzytometermessung von 4G1-Zellen, die mit Ligand ohne Antikörper inkubiert wurden, und das Histogramm B einer Durchflußzytometermessung von 4G1-Zellen, die mit Ligand und Antikörper BV10A4H2 (dort mit BV10 bezeichnet) inkubiert wurden; und

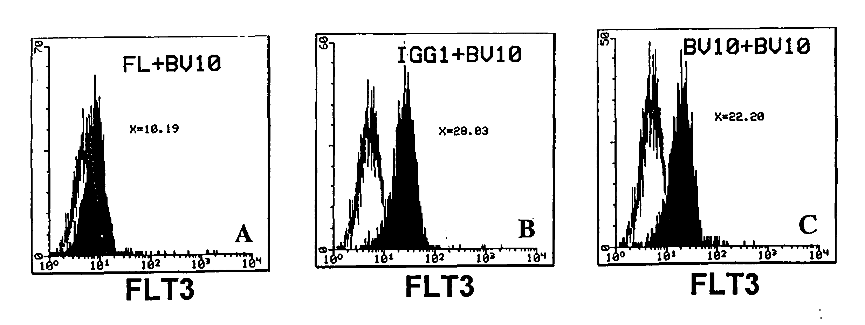

- Fig. 2: umfaßt drei Histogramme von Durchflußzytometermessungen an BV-173-Zellen, die bei B mit einem Kontrollantikörper vorinkubiert und mit dem Antikörper BV10A4H2 (dort BV10) und dem Ligand nachinkubiert wurden, bei C direkt mit dem erfindungsgemäßen Antikörper BV10A4H2 (dort BV10) und dem Ligand inkubiert wurden und bei A allein mit dem Ligand inkubiert wurden.

### Beispiel 1: Herstellung und Charakterisierung von monoklonalen Antikörpern gegen das FLT3/FLK2-Rezeptorprotein

Als Antigen werden Zellen der hämatopoetischen Pro-B-Zellinie BV-173 (DSM ACC20) verwendet.

Acht Wochen alte Balb/c-Mäuse werden zweimal in Intervallen von 10 Tagen intraperitoneal mit 10⁷ Zellen der Zellinie BV-173 immunisiert. Vier Tage vor der Fusion werden 5 x 10⁵ Zellen direkt in die Milz appliziert, um die Immunantwort zu verstärken.

Die Antikörper-Bildung im Mausorganismus wird dadurch überprüft, daß das Blutserum des betreffenden Tieres in dem dem Fachmann geläufigen ELISA-Test auf Bindungseigenschaften mit dem Antigen untersucht wird.

Nach ca. 3 Wochen werden die Lymphozyten des erfolgreich immunisierten Tieres gewonnen, indem die Milz herausoperiert und zu einer Zellsuspension zerkleinert wird.

Die suspendierten Milzzellen werden in Anwesenheit von Polyethylenglykol mit Myelomzellen des bekannten Stammes SP2/0 fusioniert. Die Fusionskultur wird in Hypoxanthin-, Aminopterin- und Thymidin-(HAT-)haltigem Medium, hier in HAT-RPMI-1640, kultiviert, in dem sich nur Hybridzellen vermehren können, da diese sowohl die Eigenschaft der Myelomzellen zur unbegrenzten Teilungsfähigkeit als auch die Eigenschaft der Antikörper produzierenden Lymphozyten zum Wachstum in HAT-haltigem Medium haben.

Nach der Fusion werden die Zellen in Napfplatten ausplattiert und bei 37 °C, 5 % Co₂ inkubiert.

Die Kulturüberstände werden nach 10-14 Tagen auf Zellen der Mauszellinie BA/F3, die zuvor mit der kompletten cDNA-Sequenz für den humanen FLT3/FLK2-Rezeptor transfiziert wurde (BA/F3-huFLT3), im Durchflußzytometer gescreent.

Um diese BA/F3-huFLT3-Zellen zu gewinnen, wurde die komplette Codesequenz der humanen cDNA für das FLT3/FLK2-Rezeptorprotein in den Säugerexpressionsvektor pCD-SRα kloniert und zusammen mit einem Plasmid, das die G418-Resistenz überträgt, in die Interleukin-3-abhängigen BA/F3-Zellen eingebracht.

Transfizierte Zellen, die den FLT3/FLK2-Rezeptor exprimieren, wurden aufgrund ihrer G418-Resistenz und anhand ihres Wachstums in Gegenwart von rekombiniertem FLT3/FLK2-Rezeptorprotein-Ligand (FL) (Lyman et al., Cell 75:1157, 1993) isoliert und angereichert. Ein Klon, 4G1, wurde nach Grenzverdünnung ausgewählt und auf seine Fähigkeit zur Expression von FLT3/FLK2-Rezeptorprotein überprüft. Dazu wurden die Zellen dieses Klons 4G1 zunächst mit radioaktivem Methionin (³⁵S) markiert und anschließend mit einem Kaninchen-Antiserum, das gegen die insertierte Domäne der Tyrosin-Kinase des FLT3/FLK2-Rezeptorproteins gerichtet ist, inkubiert. Das dadurch erhaltene Immunpräzipitat wurde mittels Polyacrylamid-Gelelektrophorese analysiert.

Die auf diese Weise als BA/F3-huFLT3 bestätigten Zellen des Klons 4G1 werden in dem Screeningverfahren als Antigen eingesetzt.

In einem Folgeschritt werden die Überstände auf Reaktivität mit Zellen der Zellinie BA/F3 getestet, da diese keine Antigene exprimieren.

Hybridome, die Antikörper mit einer Spezifität für 4G1-Zellen produzieren, werden ausgewählt und nach dem bekannten Grenzverdünnungsverfahren vereinzelt und kultiviert, d.h. kloniert.

Positiv reagierende Hybridomzellkulturen werden weiter kultiviert, die Antikörper angereichert, gereinigt und charakterisiert.

Nach der obigen Screening-Strategie wurde der monoklonale Antikörper BV10A4H2 erhalten. Der IgG1 Isotyp wurde über ein Phycoerythrin (PE)-konjugiertes Anti-Isotyp-spezifisches Antiserum durch direkte Immunfluoreszenz mittels Durchflußzytometrie bestimmt.

Die Produktion, Reinigung und Charakterisierung der Antikörper erfolgte mit den in Fachkreisen allgemein bekannten Methoden. Der Antikörper BV10A4H2, der von den unter der Nummer DSM ACC2248 bei der Deutschen Sammlung von Mikroorganismen und Zellkulturen GmbH (DSM) hinterlegten Hybridomzellen erzeugt wird, weist die folgenden charakteristischen Merkmale auf:
- Immunglobulinklasse:: IgG1
- spezifische Bindungsaffinität an:: humanes FLT3/FLK2-Rezeptorprotein (extralzelluläre Domäne)

### Beispiel 2: Identifizierung des von dem monoklonalen Antikörper BV10A4H2 erkannten Antigens.

Die Identifizierung des Antigens erfolgte durch Bindungsversuche an Zellen mit und ohne FLT3/FLK2-Rezeptor.

Eine Probe (A) mit Mauszellen der gemäß Beispiel 1 erzeugten Zellinie BA/F3-huFLT3, die mit der humanen cDNA für den FLT3/FLK2-Rezeptor transfiziert sind, wurde mit dem erfindungsgemäßen Antikörper BV10A4H2 inkubiert.

Zur Kontrolle wurde eine Probe (B) mit Mauszellen der nicht transfizierten Zellinie BA/F3 ebenfalls mit dem Antikörper BV10A4H2 in derselben Konzentration wie Probe (A) inkubiert.

Beide Proben wurden mit einem anti-IgG1-PE-Antiserum markiert und anschließend im Durchflußzytometer analysiert.

Eine Bindung des Antikörpers BV10A4H2 konnte ausschließlich bei Probe (A) nachgewiesen werden. Das heißt, daß der erfindungsgemäße Antikörper BV10A4H2 spezifisch an diejenigen Zellen gebunden hat, die durch die cDNA für humanen FLT3/FLK2-Rezeptor transformiert wurden und infolgedessen den humanen FLT3/FLK2-Rezeptor exprimieren.

### Beispiel 3: Identifizierung des monoklonalen Antikörpers BV10A4H2 als nicht antagonistisch und leicht agonistisch wirkender Antikörper

Die durch die Bindung des Liganden initiierte Aktivierung des Rezeptors führt bei Tyrosin-Kinase-Rezeptoren, wie dem FLT3/FLK2-Rezeptor zunächst zur Bildung von Rezeptordimerkomplexen und deren Internalisierung in die Zelle. Manche Antikörper können den Liganden simulieren, d.h. sie wirken agonistisch, und führen deshalb ebenfalls zu einer solchen Rezeptordimer-Bildung und -Internalisierung (Bühring et al., Cancer Res. 53: 4424, 1993).

Eine Probe (A) 4G1-Zellen (gemäß Beispiel 1 gewonnen) wurde mit 200 ng/ml biotyniliertem FLT3 Ligand-Biotin und Streptavidin-Phycoerythrin (SA-PE) inkubiert.

Eine zweite Probe 4G1-Zellen (B) wurde zunächst 30 Minuten mit 7µg/ml Antikörper BV10A4H2 inkubiert und anschließend wie Probe (1A) behandelt.

Beide Proben wurden im Durchflußzytometer analysiert. Die dabei erhaltenen Histogramme sind in Fig. 1 dargestellt.

Beide Histogramme zeigen gleichstarke Signale. Das heißt, daß der erfindungsgemäße Antikörper BV10A4H2 keinen hemmenden Einfluß auf die Bíndung des Liganden hat und somit nicht antagonistisch wirkt (Bühring et al., Cancer Res 53:4424, 1993).

Zum Nachweis der agonistischen Reaktion wurde eine Probe (B) mit Zellen der hämatopoetischen Pro-B -Zellinie BV 173 (DSM ACC 20; frei verfügbar) 2 Std. bei 37°C mit einem Kontroll-IgG1-Antikörper vorinkubiert, dann mit dem erfindungsgemäßen Antikörper BV10A4H2 inkubiert und schließlich mit anti-IgG1-PE-Antiserum nachinkubiert.

Eine weitere Probe (C) wurde zuerst mit dem Antikörper BV10A4H2 inkubiert und anschließend wie Probe (B) behandelt.

Eine Kontrollprobe (A) wurde zuerst mit 100 ng/ml FLT3/FLK2-Rezeptor-Ligand inkubiert und dann wie Probe (B) behandelt.

Alle drei Proben wurden im Durchflußzytometer analysiert. Die dabei erhaltenen Histogramme sind in Fig. 2 dargestellt. Das Histogramm C zeigt ein gegenüber dem Histogramm B um ca. 33% vermindertes Signal. Das heißt, daß der erfindungsgemäße Antikörper BV10A4H2 die Internalisierung von einigen FLT3/FLK2-Rezeptoren bewirkt hat und damit leicht agonistisch (den Liganden simulierend), wirkt.

Das Histogramm A zeigt gegenüber dem Histogramm B, bedingt durch die naturgemäße Liganden induzierte Internalisierung der FLT3/FLK2-Rezeptoren, ein um 90% vermindertes Signal.

### Beispiel 4: Verwendung des monoklonalen Antikörpers BV10A4H2 zum Nachweis von Subpopulationen der Blutstammzellen

Eine Probe frisch gewonnener Knochenmarkzellen wurde im Ficoll-Hypaque-Dichtegradienten aufgetrennt und die normalen, mononuklearen Knochenmarkzellen isoliert und angereichert.

Diese mononuklearen Knochenmarkzellen wurden dann mit einem PE-Konjugat des erfindungsgemäßen Antikörpers BV10A4H2 sowie mit bekannten Fluorescein-Isothiocyanat (FITC)-konjugierten Antikörpern gegen die Antigene HLA-DR, CD34 bzw. CD62L von Stammzellen, CD33 von myeloiden Zellen, CD71 von erythroiden und Vorläuferzellen, CD10 bzw. CD19 von B-Zellen und CD3 bzw. CD7 von T-Zellen inkubiert.

Die Zellen wurden im Durchflußzytometer analysiert.

Die Analyseergebnisse zeigen:
- eine starke Koexpression von FLT3/FLK2 mit den Stammzellmarkern CD34, HLA-DR und CD62L (55% bzw. 100% bzw. 60%),
- eine schwächere Koexpression mit dem myeloischen Zellmarker CD33 und den erythroiden und Vorläuferzellmarkern CD10 und CD19 (60% bzw. 20% bzw. 20%), und
- keine Koexpression mit den T-Zellmarkern CD7 und CD3.

Das bedeutet, daß mit dem erfindungsgemäßen Antikörper BV10A4H2 Blutstammzellen sowie myeloide Zellen und Vorläuferzellen der B-Reihe identifiziert und insbesondere von T-Zellen unterschieden werden können.

Zur Charakterisierung von Subpopulationen der CD34⁺-Stammzellpopulation wurde eine 3-Farben-Analyse durchgeführt:

Hierzu wurden die mononuklearen Zellen mit einem PE-Konjugat des erfindungsgemäßen Antikörpers BV10A4H2, einem bekannten FITC-konjugierten Antikörper gegen CD34 und einem bekannten Biotin-konjugierten Antikörper gegen CD117 inkubiert und schließlich im Durchflußzytometer analysiert.

Die erhaltenen Ergebnisse zeigen, daß 23% der CD34⁺-Zellen auch das Rezeptorprotein FLT3/FLK2 eryrimieren und 50% der Zellen dieser Subpopulation auch das Protein CD117 aufweisen.

Untersuchungen an CD34⁺⁺- und CD34⁺⁺⁺-Zellen zeigen, daß die Subpopulation der FLT3/FLK2⁺ / CD117⁺-Zellen mit fallender CD34-Expression (von CD34⁺⁺⁺ über CD34⁺⁺ bis zu CD34⁺), das heißt mit fortschreitender Entwicklung der Blutstammzellen, zahlenmäßig abnimmt, während die Subpopulation der FLT3/FLK2⁺ / CD117 ⁻-Zellen anwächst.

Mit dem erfindungsgemäßen Antikörper BV10A4H2 ist es somit möglich, zwei neue Subpopulationen der CD34-positiven Population der Blutstammzellen zu isolieren, nämlich die Subpopulation FLT3/FLK2⁺ / CD117⁺ und die Subpopulation FLT3/FLK2⁺ / CD117 ⁻.

### Beispiel 5: Verwendung des monoklonalen Antikörpers BV10A4H2 zum Nachweis von leukämischen Blasten der myeloischen und B-lymphozytären Reihe

Aus Knochenmark oder peripherem Blut von Leukämie-Patienten wurde eine reine (>90%) Population von leukämischen Blasten mittels Zentrifugation in einem Ficoll-Gradienten gewonnen.

Die Zellen wurden gemäß der Französisch-Amerikanisch-Britischen (FAB)-Klassifikation (Bennet et al., Ann. Intern. Med. 103:620, 1985) klassifiziert und diese Klassifizierung durch phänotypische Analysen bestätigt.

Die Zellen wurden mit dem Antikörper BV10A4H2 inkubiert, der gebundene Antikörper mit einem PE-konjugierten Anti-IgG1-Antiserum markiert und die Zellen im Durchflußzytometer analysiert.

In den meisten Blasten-Populationen von Patienten mit akuter myeloischer und/oder B-lymphozytärer Leukämie wurde eine positive Reaktion und damit das Vorhandensein von FLT3/FLK2-Rezeptorprotein nachgewiesen.

Ergänzend zu diesen Untersuchungen an frisch isolierten Leukämiezellen wurden Zellproben von megakaryoblastischen Zellinien, myeloid/monocytischen Zellinien und B-Zellinien getestet.

Die Zellen sämtlicher Proben wurden ebenfalls mit dem Antikörper BV10A4H2 inkubiert, der gebundene Antikörper mit einem PE-konjugierten Anti-IgG1-Antiserum markiert und die Zellen im Durchflußzytometer analysiert.

Eine positive Reaktion und damit eine Expression von FLT3/FLK2-Rezeptorprotein wurde auf den meisten Zellinien der B-lymphozytären Reihe und auf wenigen der myeloischen Reihe gefunden.

Der erfindungsgemäße Antikörper BV10A4H2 erweist sich somit als besonders geeignetes Nachweismittel für die Identifizierung von malignen hämatopoetischen Zellen der myeloischen und B-lymphozytären Reihe.

## Patentansprüche

1. Monoklonaler Antikörper, der spezifisch an das humane FLT3/FLK2-Rezeptorprotein bindet.

2. Monoklonaler Antikörper nach Anspruch 1, dadurch gekennzeichnet, daß er von Hybridomzellen produziert und freigesetzt wird, die am 19. Dezember 1995 unter der Nummer DSM ACC2248 bei der Deutschen Sammlung von Mikroorganismen und Zellkulturen GmbH (DSM) gemäß dem Budapester Vertrag hinterlegt worden sind und die Bezeichnung BV10A4H2 tragen.

3. Hybridomzellen, dadurch gekennzeichnet, daß sie die Fähigkeit aufweisen, einen Antikörper gemäß einem der Ansprüche 1 oder 2 zu erzeugen.

4. Hybridomzellen nach Anspruch 3, dadurch gekennzeichnet, daß sie am 19. Dezember 1995 unter der Nummer DSM ACC2248 bei der Deutschen Sammlung von Mikroorganismen und Zellkulturen GmbH, DSM, gemäß dem Budapester Vertrag hinterlegt worden sind und die Bezeichnung BV10A4H2 tragen.

5. Verfahren zur Herstellung von Hybridomzellen, die einen Antikörper nach einem der Ansprüche 1 oder 2 freisetzen, mit den Schritten:
1. Immunisierung oder Sensibilisierung eines Tieres, vorzugsweise einer Maus vom Balb/c-Stamm, mit dem Antigen bzw. Immunogen;
2. Gewinnung der antikörperproduzierenden Zellen, vorzugsweise der Milzlymphozyten dieses Tieres;
3. Fusionierung dieser antikörperproduzierenden Zellen mit einer stabilen, immortalisierten Zellinie, vorzugsweise einer Myelomzellinie, zu Hybridomzellen; und
4. Vereinzelung und Vermehrung (Klonierung) solcher Hybridomzellen, die einen Antikörper sezernieren, der an das Antigen bindet,
dadurch gekennzeichnet, daß das Tier mit Zellen der häamtopoetischen Pro-B-Zellinie BV-173 (DSM ACC20) immunisiert wird.

6. Verfahren nach Anspruch 5, dadurch gekennzeichnet, daß bei der Vereinzelung solche Hybridomzellen ausgewählt werden, die Antikörper mit einer Spezifität für Zellen der Mauszellinie BA/F3, die zuvor mit einer cDNA für den humanen FLT3/FLK2-Rezeptor transfiziert wurden, produzieren.

7. Verwendung eines Antikörpers nach einem der Ansprüche 1 oder 2 zur diagnostischen Behandlung von Tumoren, insbesondere von malignen hämatopoetischen Zellen.

8. Verwendung eines Antikörpers nach einem der Ansprüche 1 oder 2 zur therapeutischen Behandlung von Tumoren, insbesondere von malignen hämatopoetischen Zellen.

9. Pharmazeutisches Mittel zur diagnostischen Behandlung von Tumoren, dadurch gekennzeichnet, daß es einen Antikörper gemäß einem der Ansprüche 1 oder 2 enthält.

10. Pharmazeutisches Mittel nach Anspruch 9, dadurch gekennzeichnet, daß der Antikörper an ein zellulär zielgerichtetes Therapeutikum gekoppelt ist.

11. Pharmazeutisches Mittel zur therapeutischen Behandlung von Tumoren, dadurch gekennzeichnet, daß es einen Antikörper nach einem der Ansprüche 1 oder 2 enthält.

12. Pharmazeutisches Mittel nach Anspruch 11, dadurch gekennzeichnet, daß der Antikörper an ein zellulär zielgerichtetes Diagnostikum gekoppelt ist.

13. Kit zum Nachweis von FLT3/FLK2-Rezeptorprotein, dadurch gekennzeichnet, daß er einen Antikörper nach einem der Ansprüche 1 oder 2 enthält.

14. Verwendung eines Antikörpers nach einem der Ansprüche 1 oder 2 zum Nachweis von hämatopoetischen Zellen.

15. Kit zum Nachweis von hämatopoetischen Zellen, dadurch gekennzeichnet, daß er einen Antikörper nach einem der Ansprüche 1 oder 2 enthält.

16. Verwendung eines Antikörpers nach einem der Ansprüche 1 oder 2 zum Nachweis von Stammzellsubpopulationen.
